# EUROPEAN PATENT APPLICATION

(11) **EP 2 065 371 A1**
(43) Date of publication of application: **03.06.2009**
(21) Application number: 07023240.0
(22) Date of filing: 30.11.2007
(51) Int. Cl.: C07D 213/81, C07C 209/52, C07B 53/00

(54) **Chiral organic catalysts for the stereoselective reduction of carbon-nitrogen double bonds for the preparation of enantiomerically enriched amines**

(71) Applicant: UNIVERSITA' DEGLI STUDI DI MILANO, 20122 Milano (IT)
(72) Inventor: Benaglia, Maurizio, 22071 Cardorago (CO) (IT); Guizzetti, Stafania, 20143 Milano (IT)
(74) Representative: Minoja, Fabrizio

(57) **Abstract**

The invention relates to compounds of general formula (I): 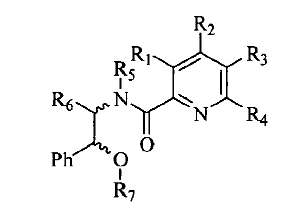 wherein R₁-R₇ are as defined in the description
for use as catalysts in the stereoselective reduction of imines in the presence of trichlorosilane as reducing agent.

## Description

### Field of the invention

The present invention relates to metal-free catalysts, in particular to picolinic acid derivatives, for use in the preparation of enantiomerically enriched amines.

### State of the art

The reduction of carbon-nitrogen double bonds is of remarkable interest in the preparation of pharmaceutical products, agrochemicals, fragrances and precursors of biologically active molecules.

Catalytic systems currently available for the enantioselective reduction of carbon-nitrogen double bonds are either organometallic compounds (Catalytic Asymmetric Synthesis, 2nd ed.: Ojima, I., Ed.: Wiley: New York, 2000 and H.U. Blaser Adv. Synth. Catal., 2002, 344, 17) or metal-free compounds.

The former are quite expensive, due to the fact that they usually consist of an enantiomerically pure ligand (whose synthesis may be costly and troublesome) and by a metal, which is often an expensive one. Organometallic catalysts may cause product metal contamination, which represents a problem especially in the case of pharmaceutical compounds and may also require particular disposal procedures.

Examples of organic catalysts are chiral binaphthol-derived phosphoric acids (Review: T. Akiyama, J. Itoh, K. Fuchibe Adv. Synth. Catal.. 2006, 348, 999) which were successfully employed in the reduction of imines and in reductive amination processes [(a) M. Rueping, E. Sugiono, C. Azap, T. Theissmann, M. Bolte Org. Lett. 2005, 7, 3781. (b) S. Hoffman, A.M. Seayad B. List Angew. Chem. Int. Ed. Engl. 2005, 44, 7424. (c) R.I. Storer, D.E. Carrera, Y. Ni, D.W.C. MacMillan J. Am. Chem. Soc. 2006, 128, 84].

These metal-free catalysts often have high molecular weight (> 600 Da) and require the Hantzsch ester as reducing agent, a dihydropyridine compound that must be separated from the reaction product.

Recently, the concept of "organic catalyst" has been introduced to indicate a class of molecules of relatively low molecular weight and simple structure, able to promote organic reactions [Reviews: (a) Dalko P. I., Moisan L., Angew. Chem. Int. Ed. Engl. 2004, 43, 5138; (b) Berkessel A., Gröger H., Asymmetric Organocatalysis; Wiley-VCH, Weinheim (Germania), 2005]; in general, these compounds do not suffer from the above-mentioned disadvantages.

Metal-free catalysts have also been developed to steroselectively reduce imines with trichlorosilane. In this case the reducing agent, trichlorosilane, is activated by coordination with a Lewis base, such as *N*,*N*-dimethylformamide, acetonitrile or a trialkylamine, to generate a hexacoordinated hydridosilicate, the actual active reducing agent that operates under mild conditions. For example, N-formyl derivatives of amino acids sometimes proved able to promote the reduction with high enantioselectivity [(a) Iwasaki, F., Onomura, O., Mishima, K., Kanematsu, T., Maki, T., Matsumura, Y., Tetrahedron Lett. 2001, 42, 2525. (b) Malkov, A. V., Ston 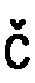 ius, S., MacDougall, K.N., Mariani, A., McGeoch, G. D., Ko 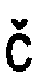 ovský P., *Tetrahedron* **2006,** *62*, 264. (c) Matsumura, Y., Ogura, K., Kouchi, Y., Iwasaki, F., Onomura, O. Org. Lett. 2006, 17, 3789], but they suffer from stability problems and in some cases they are expensive. Recently, other chiral Lewis bases in the form of *N*-formamides were prepared from (S)-pipecolinic acid and enantiopure 2-amino-1,2-diphenylethanol, both commercially available and successfully employed in the reduction of ketoimines [(a) Wang, Z., Ye, X., Wei, S., Wu, P., Zhang, A., Sun, J. Org. Lett. 2006, 5, 999. (b) L. Zhou, Z. Wang, S. Wei, J. Sun Chem. Commun. 2007, 2977]. Also quinoline, isoquinoline and pyridine-derived chiral oxazolines may be efficient promoters for the addition of trichlorosilane to ketones and imines (Malkov, A.V., Liddon, A.J.P.S., Ramírez-López, P., Bendová, L., Haigh, D., Ko 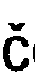 ovský, P. *Angew. Chem. Int. Ed.* **2006,** *45*, 1432). Very recently a new family of organocatalysts able to activate trichlorosilane have been disclosed. The stereoselective reduction of a broad range of *N*-aryl ketimines was catalyzed by a chiral sulfinamide, which contains a stereogenic sulfur atom (Pei, D., Wang, Z., Wei, S., Zhang, Y., Sun, J. Org. Lett. 2006, 25, 5913). However, many of these catalysts are poorly stable, difficult to prepare and their chemical and stereochemical efficiency is variable. Finally, it has been reported that N-picolinoylpyrrolidine derivatives activate trichlorosilane in the reduction of aromatic imines (Onomura, O., Kouchi, Y., Iwasaki, F., Matsumura,Y. Tetrahedron Lett. 2006, 47, 3751).

### Description of the invention

It has now been found that compounds of general formula **(I)** 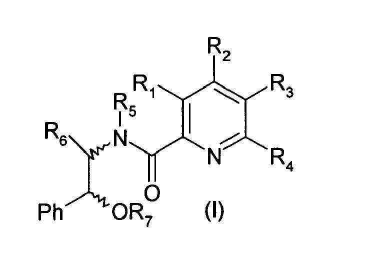 wherein:
R₁ is H, halogen, preferably Cl, Br, I or (C₁-C₄)straight or branched alkyl, preferably Me;
R₂ is H or halogen, preferably Cl or Br;
R₃ is H or halogen, preferably Cl;
R₄ is H or halogen, preferably Cl;
R₅ is (C₁-C₄)straight or branched alkyl, preferably Me or *n*-Pr, or benzyl, optionally substituted with one or more halogens or (C₁-C₄)straight or branched alkyl groups, which can be the same or different from one another;
R₆ is (C₁-C₄)straight or branched alkyl, preferably Me or *i*-Pr, or R₆ can be linked to the adjacent 1-phenyl group to form a ring of formula A: 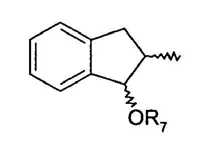
and R₇ is H, (C₁-C₄)straight or branched alkyl, preferably Me, or a CO-aryl group, wherein aryl is selected from phenyl, naphthyl, thiophenyl, furyl, oxazolyl, thiazolyl and pyridyl and is preferably 2-pyridyl
efficiently promote the reduction of ketoimines in the presence of trichlorosilane as reducing agent.

Preferred compounds of formula **(I)** are those wherein R₅ and R₆ are methyl and R₇ is hydrogen.

The compounds of the invention can be prepared by reaction of the picolinic acid derivative of formula **(II)** 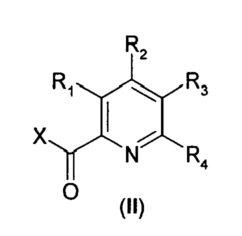 wherein R₁-R₄ are as defined above and X is a carboxy-activating group, such as chlorine or bromine
and an aminoalcohol of formula **(III)** 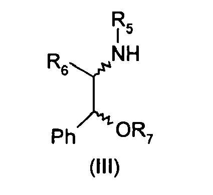 wherein R₅-R₇ are as defined above
in the presence of a suitable organic solvent, preferably dichloromethane or chloroform, more preferably chloroform, usually at room temperature.

The compound of formula **(II)** is usually prepared *in situ* from a picolinic acid of formula **(IV)** 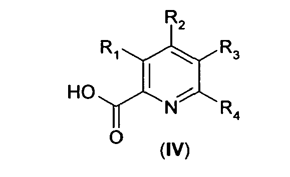 wherein R₁-R₄ are as defined above by reaction with a halogenating agent in the presence of a dehydrating agent.

According to a further embodiment, the compounds of the invention are prepared by reaction of the picolinic acid **(IV)** and the amino-alcohol **(III)** in the presence of a dehydrating agent.

In particular, the following compounds of formula **(I)** have been prepared:

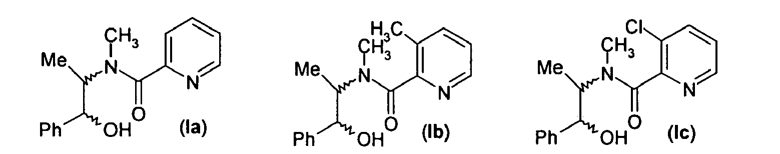

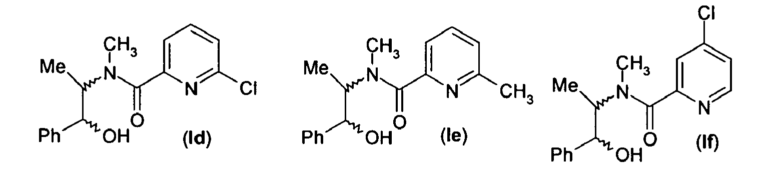

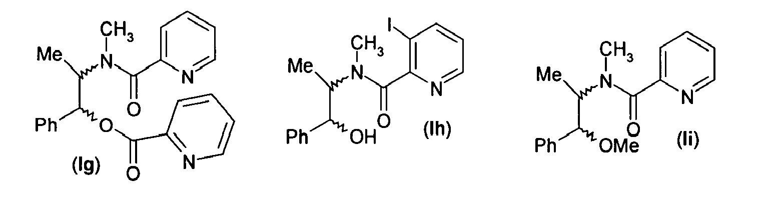

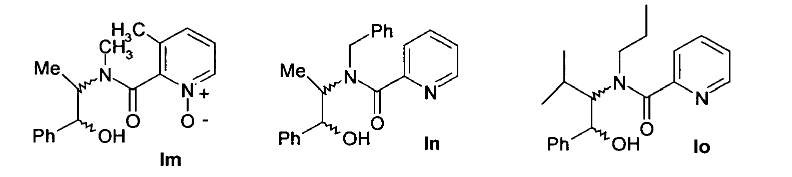

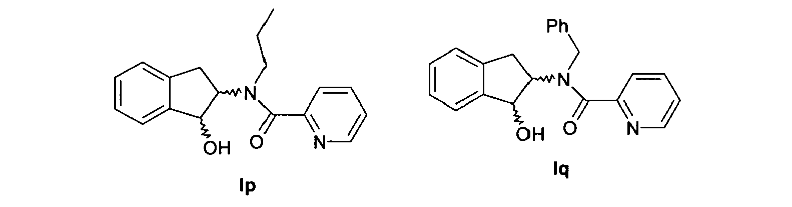

The compounds of the invention catalyze in particular the reduction of imines of formula **(V)** 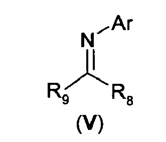 wherein:
Ar is phenyl, optionally substituted with one or more straight or branched (C₁-C₄)alkyl or (C₁-C₄)alkoxy groups, which can be the same or different from one another;
R₈ is selected from straight or branched (C₁-C₄)alkyl, straight or branched (C₁-C₄)alkoxy and COOR₁₀ wherein R₁₀ is straight or branched (C₁-C₄)alkyl;
R₉ is selected from straight or branched (C₁-C₄)alkyl, phenyl and naphthyl optionally substituted with one or more straight or branched (C₁-C₄)alkyl or (C₁-C₄)haloalkyl groups, which can be the same or different from one another.

Therefore, object of the present invention is also a process for the reduction of imines of formula **(V)** to amines of formula **(VI)** 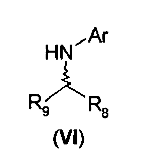 wherein R₈ and R₉ are as defined above
in the presence of a compound of formula **(I)** as catalyst and trichlorosilane as reducing agent.

The reduction reaction is usually carried out in a chlorinated organic solvent, preferably dichloromethane or chloroform, more preferably chloroform, using from 1 to 10% of compound **(I)** and from 1.5 to 3 mol/eq of trichlorosilane with respect to the imine **(V).** The reaction is usually carried out at a temperature ranging from room temperature to -20°C. It has been found that the reduction reaction occurs with high yields and high enantiomeric excess also in the presence of 1% of compound **(I);** mixtures of compounds of formula **(I)** can also be used. The reaction can be carried out also preparing the imine of formula **(I)** in situ from a ketone of formula R₈COR₉ and an amine of formula Ar-NH₂, wherein Ar is as defined above so as to accomplish a reductive amination process.

The compounds of the invention are advantageous in that they can be easily prepared from commercially available products, i.e. ephedrine or a derivative thereof and picolinic acid and in that they are stable in comparison with organometallic catalysts. Furthermore, they catalyse the reduction under mild conditions; the resulting chiral amine can be isolated from the reaction mixture by a simple aqueous workup and the compound of formula **(I)** can be recovered at the end of the reaction by flash chromatography on silica gel.

These advantages will be clearer from the following experimental section, which illustrates the invention in greater detail.

### EXPERIMENTAL SECTION

### 1. Preparation of the catalysts

### Example 1.1 - Synthesis of diastereoisomer (1R.2S) of compound (Ia)

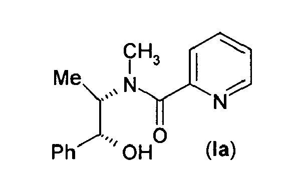

Picolinic acid, EDC (1-ethyl-3-(3-dimethylaminopropyl)carbodiimide) and HOBt (1-hydroxy 1H-benzotriazole) were dissolved in CHCl₃ under dry atmosphere and stirred; after 15 minutes ephedrine was added. Table 1 below indicates the amount of each reagent.

**Table 1**

| | PM (g/mol) | q (mg) | eq | mmol | V |
|---|---|---|---|---|---|
| Picolinic acid | 123,11 | 150 | 1,0 | 1,22 | |
| (1*R*,2*S*) Ephedrine | 165.23 | 242 | 1,2 | 1,46 | |
| EDC | 191,70 | 280 | 1,2 | 1,46 | |
| HOBt | 135,13 | 198 | 1,2 | 1,46 | |
| CHCl₃ dry | | | | | 10 ml |

After stirring for 24 hours at 25°C the solvent was evaporated under reduced pressure and the product was purified by flash chromatography (d = 2.5 cm, h = 18 cm, eluent: CH₂Cl₂/MeOH 98:2).
Yield = 70%
MW = 270.33 g/mol 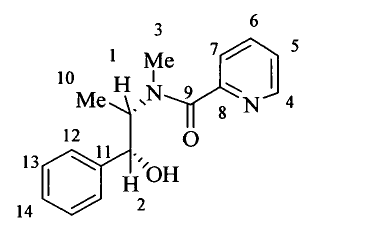 Rotamer 1:
¹H-NMR (300 MHz, CDCl₃): 1 (δ 4.35, 1H); 2 (δ 4.81,1H); 3 (δ 2.80,3H); 4 (δ 8.5, 1H); 5 (δ 7.36, 1H); 6 (δ 7.75, 1H); 7 (δ 7.49, 1H); 10 (δ 1.33, 3H); 12,13,14 (δ 7.25-7.30, 5H).
¹³C-NMR (300 MHz, CDCl₃): 1 (δ 58.3, 1C); 2 (δ 76.1, 1C); 3(δ 29.7, 1C); 4 (δ 147.3, 1C); 5 (δ 124.5, 1C); 6 (δ 137.4, 1C); 7 (δ 123.1, 1C); 8 (δ 154.2, 1C); 9 (δ 169, 1C); 10 (δ 14.5, 1C); 11 (δ 141.5, 1C); 12(δ 126.7, 2C); 13 (δ 128.2, 2C); 14 (δ 127.4, 1C).
Rotamer 2:
¹H-NMR (300 MHz, CDCl₃): 1 (δ 4.57, 1H); 2 (δ 5.09,1H); 3 (δ 2.87,3H); 4 (δ 8.5, 1H); 5 (δ 7.36, 1H); 6 (δ 7.75, 1H); 7 (δ 7.49, 1H); 10 (δ 1.28, 3H); 12,13,14 (δ 7.25-7.30, 5H).
¹³C-NMR (300 MHz, CDCl₃): 1 (δ 58.6, 1C); 2 (δ 76.4, 1C); 3 (δ 35.0, 1C); 4 (δ 148.3, 1C); 5 (δ 124.5, 1C); 6 (δ 137.1, 1C); 7 (δ 124.3, 1C); 8 (δ 154.8, 1C); 9 (δ 169.8, 1C); 10 (δ 11.2, 1C); 11 (δ 142.1, 1C); 12(δ 126.3, 2C); 13 (δ 128.2, 2C); 14 (δ 127.1, 1C).
MS-ESI⁺: *mlz* 271 [M+H]⁺, *mlz =* 293 [M+Na]⁺.
[α]²³ -19 (c 0.55 in DCM);
IR (DCM): ν_{C=O} = 1725.01 cm⁻¹

### Example 1.2 - Synthesis of diastereoisomer (1S, 2S) of compound (1a)

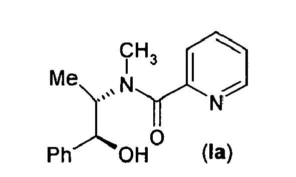

This compound was prepared following the procedure of example 1, using (1*S*, 2*R*) ephedrine. 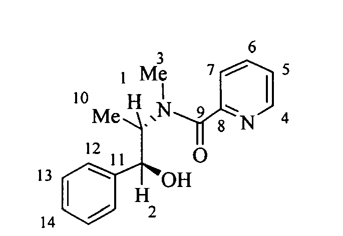 ¹H-NMR: 1 (δ 4.21, 1H); 2 (δ 4.60,1H); 3 (δ 3.18,3H); 4 (δ 8.70, 1H); 5 (δ 7.50, 1H); 6 (δ 7.94, 1H); 7 (δ 7.89, 1H); 10 (δ 0.95, 3H); 12 (δ 7.28, 2H); 13 (δ 7.34, 2H); 14 (δ 7.28, 1H).
¹³C-NMR: 1 (δ 59.0, 1C); 2 (δ 75.4, 1C); 3 (δ 27.3, 1C); 4 (δ 148.4, 1C); 5 (δ 125.2, 1C); 6 (δ 138.4, 1C); 7 (δ 125.7, 1C); 8 (δ 153.9, 1C); 9 (δ 168.6, 1C); 10 (δ 16.4, 1C); 11 (δ 143.2, 1C); 12 (δ 126.8, 2C); 13 (δ 128.5, 2C); 14 (δ 127.8, 1C).
m.p..: 108-110°C
MS-ESI⁺: *mlz* 271 [M+H]+, *mlz* = 293 [M+Na]⁺.
[α]²³ 240 (c 0.16 in DCM);

### Example 1.3 - Synthesis of compound (Id)

This compound was prepared following the procedure of example 1. 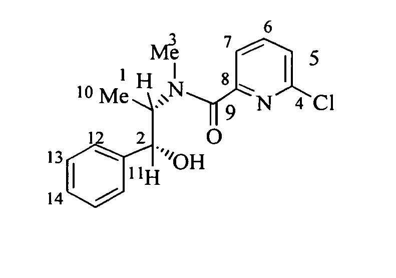 Rotamer 1:
¹H-NMR: 1 (δ 4.2, 1H); 2 (δ 4.93,1H); 3 (δ 3.0,3H); 5 (δ 7.38, 1H); 6 (δ 7.68, 1H); 7 (δ 7.15, 1H); 10 (δ 1.33, 3H); 12 (δ 7.26, 2H); 13 (δ 7.31, 2H); 14 (δ 7.31, 1H).
¹³C-NMR: 1 (δ 58.9, 1C); 2 (δ 76.3, 1C); 3 (δ 29.3, 1C); 4 (δ 150.2, 1C); 5 (δ 125.1, 1C); 6 (δ 139.7, 1C); 7 (δ 122.5, 1C); 8 (δ 154.8, 1C); 9 (δ 167.8, 1C); 10 (δ 13.7, 1C); 12 (δ 126.35, 2C); 13 (δ 128.3, 2C); 14 (δ 127.65, 1C); 11 (δ 141.6, 1C).
Rotamer 2:
¹H-NMR: 1 (δ 4.54, 1H); 2 (δ 5.08,1H); 3 (δ 2.9, 3H); 5 (δ 7.38, 1H); 6 (δ 7.75, 1H); 7 (δ 7.41, 1H); 10 (δ 1.35, 3H); 12 (δ 7.47, 2H); 13 (δ 7.38, 2H); 14 (δ 7.31, 1H).
¹³C-NMR: 1 (δ 59, 1C); 2 (δ 77, 1C); 3 (δ 35.2, 1C); 4 (δ 149.6, 1C); 5 (δ 125.1, 1C); 6 (δ 139.7, 1C); 7 (δ 121.9, 1C); 8 (δ 154.9, 1C); 9 (δ 167.4, 1C); 10 (δ 11.4, 1C); 12 (δ 126.3, 2C); 13 (δ 128.4, 2C); 14 (δ 128 1C); 11 (δ 141.8, 1C).
MS-ESI⁺: *m*/*z* = 327 [M+Na]⁺.
[α]²³ -13 (c 0.55 in DCM);

### Example 1.4 - Synthesis of compound (Ib)

This compound was prepared following the procedure of example 1. 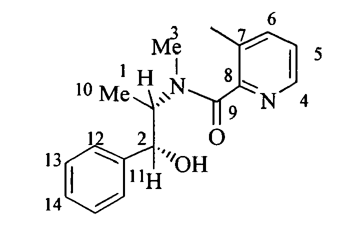 Rotamer 1:
¹H-NMR: 1 (δ 4.75, 1H); 2 (δ 5.05,1H); 3 (δ 2.67,3H); 4 (δ 8.29, 1H); 5 (δ 7.17, 1H); 6 (δ 7.50, 1H); 10 (δ 1.28, 3H); 12 (δ 7.47, 2H); 13 (δ 7.34, 2H); 14 (δ 7.25, 1 H); 7 (δ 2.09,3H).
¹³C-NMR: 1 (δ 56.5, 1C); 2 (δ 76, 1C); 3 (δ 33, 1C); 4 (δ 146.3, 1C); 5 (δ 123.6, 1C); 6 (δ 138.6, 1C); 7 (δ 130, 1C); 8 (δ 154, 1C); 9 (δ 169, 1C); 10 (δ 11.3, 1C); 12 (δ 126.4, 2C); 13 (δ 128.2, 2C); 14 (δ 127.4, 1C); 11 (δ 142, 1C); 7 (δ 17.1, 1C).
Rotamer 2:
¹H-NMR: 1 (δ 3.75, 1H); 2 (δ 4.84,1H); 3 (δ 2.98, 3H); 4 (δ 8.33, 1H); 5 (δ 7.2, 1H); 6 (δ 7.50, 1H); 10 (δ 1.2, 3H); 12 (δ 7.07, 2H); 13 (δ 7.2, 2H); 14 (δ 7.25, 1H); 7 (δ 2.06,3H).
¹³C-NMR: 1 (δ 58.9, 1C); 2 (δ 76, 1C); 3 (δ 28.5, 1C); 4 (δ 145.7, 1C); 5 (δ 123.7, 1C); 6 (δ 138.8, 1C); 7 (δ 132, 1C); 8 (δ 154, 1C); 9 (δ 169, 1C); 10 (δ 13.1, 1C); 12 (δ 126.1, 2C); 13 (δ 128.2, 2C); 14 (δ 127.5, 1C); 11 (δ 142, 1C) 7 (δ 18, 1C).
MS-ESI⁺: *m*/*z* 285 [M+H]⁺,
[α]²³ -23.7 (c 0.4 in DCM).

### Example 1.5 - Synthesis of compound (li)

This compound was prepared following the procedure of example 1. 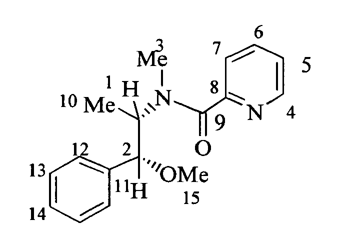 Rotamer 1:
¹H-NMR: 1 (δ 4.18, 1H); 2 (δ 4.36,1H); 3 (δ 3.12,3H); 4 (δ 8.59, 1H);5 (δ 7.29, 1H); 6 (δ 7.68, 1H); 7 (δ 7.08, 1H); 10 (δ 1.36, 3H); 12 (δ 7.43, 2H); 13 (δ 7.3, 2H); 14 (δ 7.38, 1H); 15 (δ 3.27, 3H).
¹³C-NMR: 1 (δ 58.6, 1C); 2 (δ 86.1, 1C); 3 (δ 28.6, 1C); 4 (δ 148, 1C); 5 (δ 124, 1C); 6 (δ 136, 1C); 7 (δ 124, 1C); 8 (δ 154, 1C); 9 (δ 169, 1C); 10 (δ 13.5, 1C); 12 (δ 126.8, 2C); 13 (δ 128.4, 2C); 14 (δ 127.9, 1C); 11 (δ 139.5, 1C); 15 (δ 57.1 1C).
Rotamer 2:
¹H-NMR: 1 (δ 4.75, 1H); 2 (δ 4.57,1H); 3 (δ 2.92, 3H); 4 (δ 8.59, 1H); 5 (δ 7.29, 1H); 6 (δ 7.75, 1H); 7 (δ 7.29, 1H); 10 (δ 1.36, 3H); 12 (δ 7.08, 2H); 13 (δ 7.3, 2H); 14 (δ 7.38, 1H); 15 (δ 3.32, 3H).
¹³C-NMR: 1 (δ 55.5, 1C); 2 (δ 86.1, 1C); 3 (δ 31.3, 1C); 4 (δ 148, 1C); 5 (δ 124, 1C); 6 (δ 136, 1C); 7 (δ 122.5, 1C); 8 (δ 154, 1C); 9 (δ 168.5, 1C); 10 (δ 11.7, 1C); 12 (δ 127.1, 2C); 13 (δ 128.4, 2C); 14 (δ 127.7 1C); 11 (δ 140 1C); 15 (δ 57.11C).
MS-ESI⁺: *m*/*z* 285 [M+H]⁺,
[α]²³ -35.3 (c 0.3 in DCM);

### 2. Reduction reactions

### General procedure for the reduction of imines

The catalyst (0.1-0.01% mol/eq) and the imine (1 mol/eq) are dissolved in the selected solvent under dry atmosphere and stirred at the proper reaction temperature. Trichlorosilane (typically 3.5 mol/eq) is then added and the reaction mixture is stirred until completion of the reaction. Then NaHCO₃ (sat. sol.) is added, the organic phase is separated, dried over sodium sulfate and evaporated under reduced pressure. If necessary, the product is purified by flash chromatography.

### Example 2.1 Synthesis of amine (VIa)

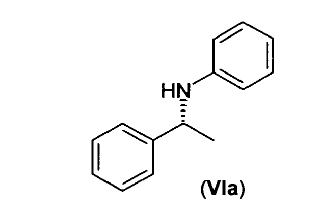

The reduction of imine **(Va)** 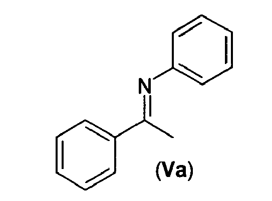 was performed in the presence of trichlorosilane (1.5 - 3 mol/eq) and a catalytic amount of compound **(Ia)** (1R, 2S) under different experimental conditions. Among the solvents tested for the reaction, the best results were obtained with chlorinated solvents (see Table 2 below).

Compound **(la)** was able to promote the reduction in dichloromethane at 25°C in quantitative yield and 69% e.e.. By lowering the reaction temperature to 0°C enantioselectivity increased to 86% without decrease of the chemical yield. By working in chloroform a further improvement in the enantioselection was observed; amine **(Va)** was isolated in 97% yield and 82% e.e. (see Table 3 below).

**Table 2**

| Entry | t (h) | Solvent | T (°C) | Yield | e.e. (%) |
|---|---|---|---|---|---|
| 1 | 15 | **CH₃CN** | 0 | 97 | **50** |
| 2 | 15 | **toluene** | 0 | 98 | **60** |
| 3 | 15 | **THF** | 0 | 94 | **48** |
| 4 | 15 | **DMF** | 0 | 94 | **0** |

**Table 3**

| Entry | t (h) | Solvent | T (°C) | Yield | e.e. (%) |
|---|---|---|---|---|---|
| 1 | 15 | **DCM** | 0 | 98 | **76** |
| 2 | 15 | **CHCl₃** | 0 | 97 | **82** |
| 3 | 15 | **DCM** | -20 | 98 | **32** |
| 4 | 15 | **CHCl₃** | -20 | 98 | **77** |
| 5 | 40 | **DCM** | -40 | 62 | **43** |
| 6 | 40 | **CHCl₃** | -40 | 32 | **43** |
| 7 | 15 | **DCM** | 25 | 98 | **69** |

A few experiments were carried out to investigate the effect of the amount of catalyst and trichlorosilane and reaction time.

The amount of catalyst can be lowered to 1% with only marginal loss (or no loss at all) of chemical efficiency and only slight decrease in enantioselectivity.

The data reported in Table 4 show that the use of an amount of catalyst of 10% allows to obtain amine **(VIa)** with 94% yield after only two hours, which is comparable with the yield obtained after 15 hours (97% yield). Even using 1% catalyst, the product was obtained in 90% yield after only 2 hours.

The amount of trichlorosilane is also an important parameter; with 1 mol/equivalent the the yield was poor, while a large excess (10 mol/eq) led to a product with lower enantioselectivity; therefore, the reactions were usually conducted using 2.5-3.5 mol/eq of trichlorosilane.

**Table 4**

| Entry | Cat loading (%) | Time (h) | Yield | e.e. (%) |
|---|---|---|---|---|
| 1 | 10 | 2 | 94 | 79 |
| 2 | 10 | 15 | 97 | 82 |
| 3 | 1 | 15 | 89 | 78 |
| 4 | 1 | 2 | 90 | 66 |
| 5 | 1 | 3,5 | 96 | 60 |

### 2.2 Synthesis of amines (VIb) and (VIc)

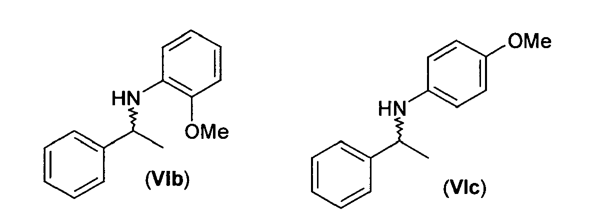

Amines **(VIb)** and **(VIc)** were prepared using 10% compound **(Ia)** as catalyst, under the best reaction conditions, in chloroform at 0 °C. The results are reported in table 5 below.

**Table 5**

| Entry | Imine | Yield (%) | e.e. (%) |
|---|---|---|---|
| 1 | **(VIb)** | 93 | 90 |
| 2 | **(VIc)** | 98 | 80 |

*N*-(2-methoxyphenyl) imine of acetophenone **(Vb)** and *N*-(4-methoxyphenyl) imine of acetophenone **(Vc)** were successfully reduced in very high yields and enantioselectivity of 90% and 80% respectively. It must be noted that amines **(VIb)** and **(VIc)** represent potential precursors of primary amine group, since the both the *ortho*-methoxyphenyl ring and the *para*-methoxyphenyl moiety can be removed.

The reduction of *N*-phenyl imine of propiophenone **(Vd)** 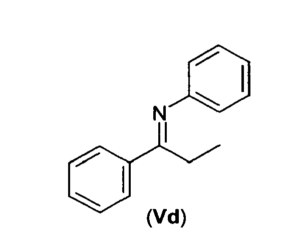 was also performed and the corresponding amine **(VId)** was obtained in 90% yield and 71% e.e.

### Example 2.3 Synthesis of amine (VIe)

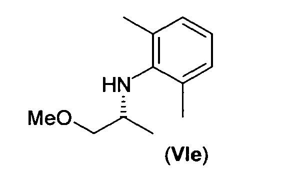

Imine **(Ve)** 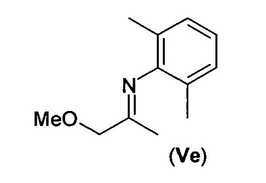 was prepared by reaction of methoxyacetone and 2,6-dimethylaniline and reduced to amine **(VIe)** (R enantiomer) with trichlorosilane in the presence of 0.1 eq. of compound **(Ia)** derived from (1*R*, 2*S*)-(-)-ephedrine, for about 15 hours. Table 6 below shows that the reaction proceeds with high yield and enantioselectivity.

**Table 6**

| Entry | Solvent | T (°C) | Yield | e.e. (%) |
|---|---|---|---|---|
| 1 | CH₂Cl₂ | 0 | 98 | 65 |
| 2 | CH₂Cl₂ | -20 | 70 | 66 |
| 3 | CHCl₃ | 0 | 79 | 70 |
| 4 | CHCl₃ | -20 | 75 | 74 |

The *S* isomer of amine **(VIe)** was prepared using the enantiomer of catalyst **(Ia)** obtained from (1*S*, 2*R*)-(+)-ephedrine, under the conditions reported in table 7.

**Table 7**

| Entry | Solvent | T (°C) | Yield | e.e. (%) |
|---|---|---|---|---|
| 1 | CHCl₃ | 0 | 98 | 70 |

### 2.4 Synthesis of amine (VIa) with different compounds of formula (Ia)

3-methyl picolinic acid was condensed with (-) ephedrine to afford compound **(Ib),** which promoted the reduction of imine **(Va)** in 98% yield and 77% e.e. in dichloromethane at 0°C (reaction time 15 hours; 0.1 eq of compound **(Ib)).**

3-chloro picolinic acid was condensed with (-) ephedrine to afford compound **(Ic),** which promoted the reduction of imine **(Va)** in 77% yield and 71% e.e. in dichloromethane at 0°C; stereoselectivity was higher at -20°C, using chloroform as solvent. The results are resumed in table 8 below (reaction time: 15 hours; 0.1 equivalents of compound **(Ia),** except for entry 5, wherein 0.01 equivalents were used).

**Table 8**

| Entry | Solvent | T (°C) | Yield | e.e. (%) |
|---|---|---|---|---|
| 1 | CH₂Cl₂ | 0 | 77 | 71 |
| 2 | CH₂Cl₂ | -20 | 91 | 75 |
| 3 | CHCl₃ | 0 | 74 | 70 |
| 4 | CHCl₃ | -20 | 95 | 77 |
| 5 | CH₂Cl₂ | 0 | 91 | 71 |

4-chloropicolinic acid was condensed with (-) ephedrine to afford compound **(If),** which promoted the reduction of imine **(Va)** in 98% yield and 83% e.e. in dichloromethane. The best results were obtained in chloroform at -20°C; the yield was basically quantitative and the enantiomeric excess was 93%. The results are resumed in table 9 below (reaction time: 15 hours; 0.1 equivalents of compound **(Ia)).**

**Table 9**

| Entry | Solvent | T (°C) | Yield | e.e. (%) |
|---|---|---|---|---|
| 1 | CHCl₃ | 0 | 98 | 87 |
| 2 | CHCl₃ | -20 | 98 | 93 |
| 3 | DCM | 0 | 98 | 83 |
| 4 | DCM | -20 | 91 | 84 |

The reduction of imine **(Va)** was also performed at -20°C in chloroform with compound **(Ia)** and the product was obtained in 91 % yield and 81 % e.e..

Table 10 reports the results of the reduction of imine **(Va)** with other compounds of formula **(I).**

**Table 10**

| Entry | Catalyst | Solvent | T (°C) | Yield | e.e. (%) |
|---|---|---|---|---|---|
| 1 | **Ig** | CH₂Cl₂ | 0 | 98 | 69 |
| 2 | **Ih** | CH₂Cl₂ | 0 | 97 | 68 |
| 3 | **Ih** | CHCl₃ | -20 | 98 | 73 |
| 4 | **li** | CH₂Cl₂ | 0 | 98 | 59 |
| 5 | **li** | CH₂Cl₂ | -20 | 98 | 66 |

### Example 2.5 Syhthesis of amines (VIf) and (VIg)

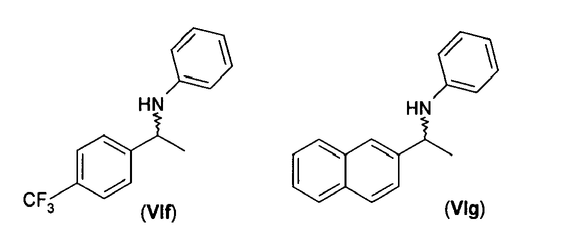

The reduction of *N*-phenyl imine of 4-trifluoromethylphenyl-methyl ketone **(Vf)** 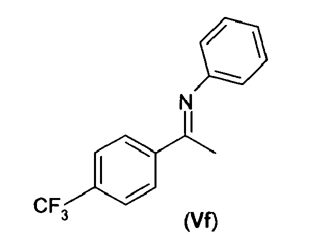 and *N*-phenyl imine of 2-naphthyl-methyl ketone **(Vg)** 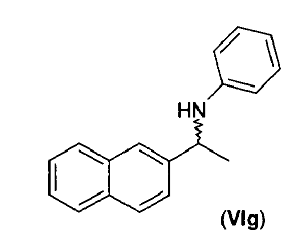 at -20°C in CHCl₃ was also successfully performed with catalyst **(Ia)** afforded the corresponding amine in 85% yield, 90% e.e. and 87% yield, 94% e.e. respectively.

## Claims

1. Compounds of general formula (I): 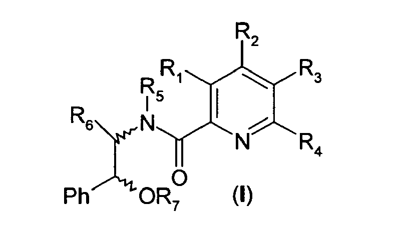 wherein:
R₁ is H, halogen or (C₁-C₄)straight or branched alkyl;
R₂ is H or halogen;
R₃ is H or halogen;
R₄ is H or halogen;
R₅ is (C₁-C₄)straight or branched alkyl or benzyl optionally substituted with one or more halogens or (C₁-C₄)straight or branched alkyl groups, which can be the same or different from one another;
R₆ is (C₁-C₄)straight or branched alkyl, or R₆ can be linked to the adjacent 1-phenyl group to form a ring of formula A: 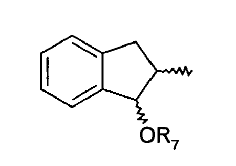
wherein R₇ is H, (C₁-C₄)straight or branched alkyl or a CO-aryl group, wherein aryl is selected from phenyl, naphthyl, thiophenyl, furyl, oxazolyl, thiazolyl and pyridyl.

2. A compound of formula (I) according to claim 1, wherein:
R₁ is H, Cl, Br, I or Me;
R₂ is H, Cl or Br;
R₃ is H or Cl;
R₄ is H or Cl;
R₅ is Me, *n*-Pr or benzyl;
R₆ is Me or *i*-Pr or R₆
and R₇ is H, Me or CO-2-pyridyl.

3. A compound of formula (I) according to claim 1 or 2 wherein R₅ and R₆ are methyl and R₇ is hydrogen.

4. A compound selected from:

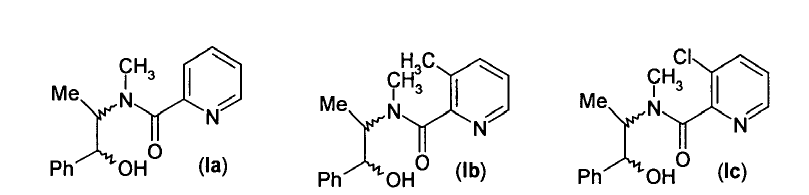

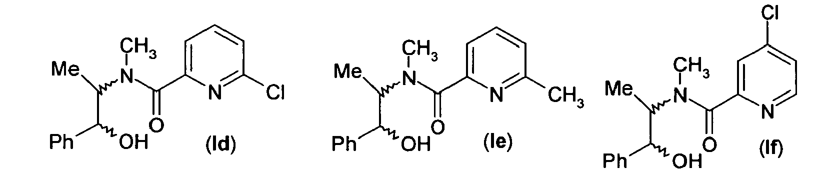

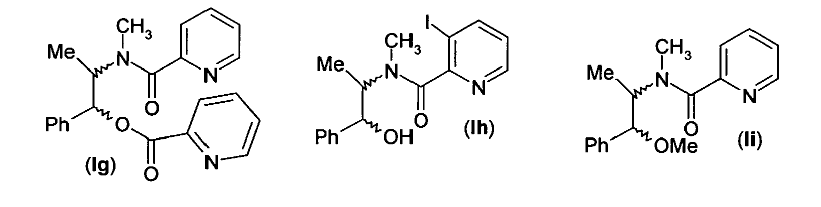

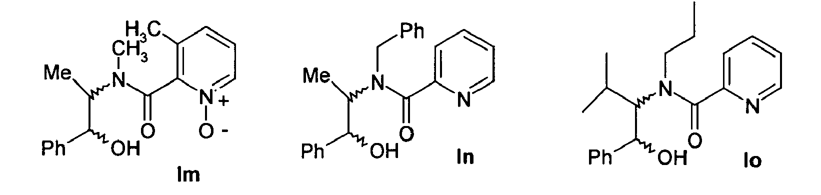

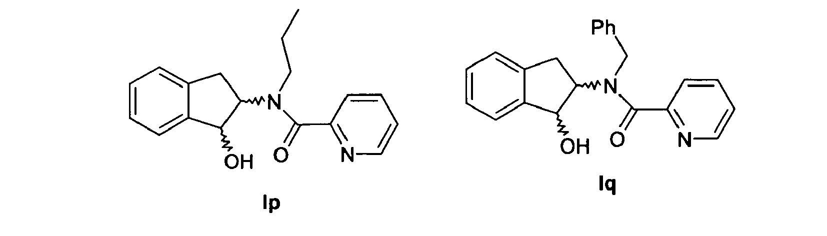

5. The use of the compounds of formula **(I)** as defined in any one of claims 1-4 as catalysts for the reduction of imines.

6. A process for the preparation of amines general formula **(VI):** 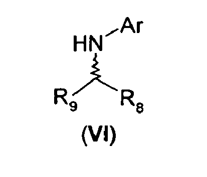 wherein:
Ar is phenyl, optionally substituted with one or more straight or branched (C₁-C₄)alkyl or (C₁-C₄)alkoxy groups, which can be the same or different from one another;
R₈ is selected from straight or branched (C₁-C₄)alkyl, straight or branched (C₁-C₄)alkoxy and COOR₁₀ wherein R₁₀ is straight or branched (C₁-C₄)alkyl;
R₉ is selected from straight or branched (C₁-C₄)alkyl, phenyl and naphthyl optionally substituted with one or more straight or branched (C₁-C₄)alkyl or (C₁-C₄)haloalkyl groups, which can be the same or different from one another.
said process comprising the reduction of imines of formula **(V):** 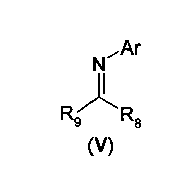 wherein Ar, R₈ and R₉ are as defined above
in the presence of a compound of formula (I) as defined in any one of claims 1-3 as catalyst, trichlorosilane as reducing agent and an organic solvent.

7. A process according to claim 6 wherein the organic solvent is chloroform or dichloromethane.

8. A process according to claim 6 or 7 wherein the compound of formula **(I)** is used in amounts of 1-10% by weight with respect to the amount of imine **(V).**

9. A process according to any one of claims 6 to 8 wherein trichlorosilane is used in amount of 1.5-3 mol/eq. with respect to imine **(V).**

10. A process according to any one of claims 6 to 8 wherein the imine of formula **(V)** is prepared *in situ* form a ketone of formula R₈COR₉ and an amine of formula ArNH₂, wherein R₈, R₉ and Ar are as defined in claim 6.
